# EUROPEAN PATENT APPLICATION

(11) **EP 0 525 885 A1**
(43) Date of publication of application: **03.02.1993**
(21) Application number: 92202248.8
(22) Date of filing: 21.07.1992
(51) Int. Cl.: C07C 323/60, C07C 317/44, A61K 31/19, A61K 31/16, A61K 31/21

(54) **New sulphur containing nonsteroidal drugs as 5-alpha reductase inhibitors**

(30) Priority: 29.07.1991 US 737088
(71) Applicant: MERCK & CO. INC., Rahway New Jersey 07065-0900 (US)
(72) Inventor: Witzel, Bruce E., Westfield, NJ 07090 (US); Tolman, Richard L., Warren, NJ 07059 (US)
(74) Representative: Thompson, John Dr.

(57) **Abstract**

Described are new non-steroidal drugs for treatment of benign prostatic hyperplasia and other disorders mediated by high 5-a reductase activity, high DHT levels and other conditions of hyperandrogenic stimulation, of the formula: 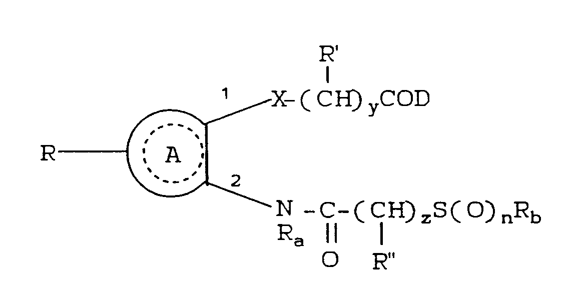
wherein A, D, X, R, R', R", Rₐ, y and z are as defined in claim 1.

## Description

### BACKGROUND OF THE INVENTION

It is well known in the art that certain undesirable physiological manifestations, such as acne vulgaris, seborrhea, female hirsutism, and male pattern baldness and particularly benign prostatic hyperplasia (BPH), art the result of hyperandrogenic stimulation caused by an excessive accumulation of dihydrotestosterone or other androgenic hormones in the metabolic system. Early attempts to provide a chemtherapeutic agent to counter the undesirable results of hyperandrogenicity resulted in the discovery of several steroidal antiandrogens having undesirable hormonal activities of their own. the estrogens, for example, not only counteract the effect of the androgens but can have a feminizing effect as well, e.g. a tendency to feminize a male host or the male fetus of a female host.

It more recently became known in the art that the principal mediator of androgenic activity in some target organs is 5a-dihydrotestosterone (DHT), and that it is formed locally in the target organ by the action of testosterone-5a-reductase or other 5a-reductase isozymes. It is known that various members of the 5a-reductase family of enzymes function to provide DHT levels in the control of various peripheral functions, i.e. male pattern baldness, beard growth, sebum production, etc. Any or all of these isozymic targets could be targets for a 5a-reductase inhibitor. It therefore has been postulated and demonstrated that inhibitors of testosterone-5a-reductase will serve to prevent or lessen symptoms of hyperandrogenic stimulation. Nayfe et al. , Steroids, 14, 269 (1969) demonstrated in vitro that methyl 4-androsten-3-one-17,8-carboxylate was a testosterone-5a-reductase inhibitor. Then Voigt and Hsia, Endocrinology, 92, 1216 (1973), Canadian Pat. No. 970,692, demonstrated that the above ester and the parent free acid, 4-androsten-3-one-17,8-carboxylic acid are both active inhibitors of testosterone-5a-reductase in vitro. They further demonstrated that topical application of either testosterone or 5a-dihydrotesterone caused enlargement of the female hamster flank organ, an androgen-dependent sebaceous structure. However, concomitant administration of 4-androsten-3- one-17,8-carboxylic acid or its methyl ester inhibited the response elicited by testosterone but did not inhibit the response elicited by 5a-dihydrotestosterone. These results were interpreted as indicating that the compounds were antiandrogenic by virtue of their ability to inhibit testosterone-5a-reductase.

Steroidal compounds particularly for the treatment of Benign Prostatic Hyperplasia (BPH) are well known in the art and constantly being developed. See the following patents: USP 4,317,817, USP 4,882,319 and EPO Publication Nos. 0 277 002, 0 343 954, 0 375 344, 0 375 345, 0 375 347, 0 375 349 (all assigned to SmithKline Beckmann Corporation) which disclose steroidal 5-alpha reductase inhibitors as BPH agents.

However, as described above, being steroids, they also can exhibit hormonal activity which may, in some cases, be undesirable, e.g. causing femininizing characteristics in men due to antiandrogen mechanisms.

Recent non-steroidal BPH active compounds have been disclosed in EP 291 245, EP 291 247 and EP 0 173 516 to ONO Pharmaceutical Co. which are outside the scope of this patent.

It is highly desired in the art to discover new non-steroidal 5-alpha reductase inhibitors which do not possess other steroidal activities with their attendant undesirable effects.

### SUMMARY OF THE INVENTION

By this invention is provided a new agent for the treatment of BPH of the following formula: 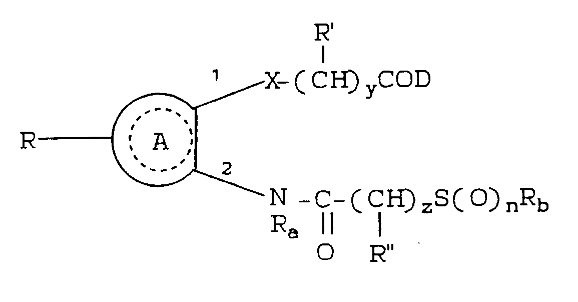
wherein
A is an 1,2-disubstituted aromatic ring, preferably a benzene ring;
D is OH, NH₂, NHR_{b}, ORb;
X is O, S, SO, or S0₂;
R is H,
C₁-C₄alkyl,
phenyl or substituted phenyl,
halo,
haloalkyl,
hydroxy,
carboxy,
cyano,
C₁-C₄ alkoxy,
C₁ -C₄ alkylthio,
C₁-C₄ alkylsulfinyl,
C₁ -C₄ alkylsulfonyl,
nitro,
amino,
C₁-C₄ mono or di-alkylamino;
R' and R" are independently H,
halo,
C₁-C₄ alkyl or C₁-C₄ alkoxy,
amino, or oxo, where CH-R' or CH-R"
in the formula become -C = O;
Rₐ is H, C₁-C₄ alkyl;
R_{b}, Rₑ are independently, C₁-C₁₂ alkyl, phenyl, phenyl-C₁-C₄ alkyl;
n is 0-2;
y is 1-6;
z is 6-20; and
wherein 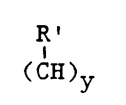
   and

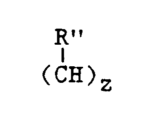

can independently represent substituted or unsubstituted alkyl radicals or alkenyl radicals containing at least one alkene bond;

and pharmaceutically acceptable salts and esters thereof.

The compounds of the instant invention are inhibitors of the human testosterone 5a-reductase.

### BRIEF DESCRIPTION OF THE INVENTION AND PREFERRED EMBODIMENTS

The scope of the compounds of the instant invention are described by the above-described formula.

In the description of the formula the following terms are used which are hereby defined:
X can be O or S, preferably one X being O, and particularly preferred wherein both Xs are O, i.e., the catechol structure.
"C₁-C₄ alkyl" includes linear or branched species, e.g. methyl, ethyl, n-propyl, isopropyl, cyclopropyl, n-butyl, isobutyl, sec-butyl, t-butyl; and "C₁-C12 alkyl" includes alkyl up to 12 carbons including n-octyl, t-decyl, n-dodecyl.
"Phenyl C1-C4 alkyl" includes benzyl, 2-phenethyl, and the like;
"C₁-C₄ alkoxy" includes linear or branched species, e.g., methoxy, ethoxy, n-propoxy, isopropoxy, n-butoxy, isobutoxy, sec-butoxy, t-butoxy;

"Halo" includes fluoro, chloro, bromo or iodo;

"Substituted phenyl" includes phenyl substituted by one or more of C₁-C₄ alkyl, C₁-C₄ alkoxy, or halo, and the like, as defined above; representative examples include o, m-, p-methoxy phenyl; 2,4-dimethoxyphenyl; 2-chloro-4-ethoxyphenyl; 3,5-dimethoxyphenyl; 2,4-dichlorophenyl; 2-bromo-4-methylphenyl, o-fluorophenyl, and the like.

"Haloalkyl" includes C1-C4 alkyl, defined above, substituted with one or more "halo" as defined above and includes: trifluoromethyl, 2,2-dichloroethyl and the like.

"C₁-C₄ alkylthio" includes C1-C4 alkyl, defined above, substituted with at least one divalent thio (-S-) grouping including; methylthio, ethylthio, isopropylthio, n-butylthio, and the like.

"C₁-C₄ alkylsulfinyl" includes C1-C4 alkyl, defined above, substituted with at least one -SO-grouping including; methylsulfinyl, ethylsulfinyl; isopropylsulfinyl, and the like.

"C₁-C₄ alkylsulfonyl" includes C1-C4 alkyl, defined above, substituted with at least one sulfonyl group, -S0₂-, including; methylsulfonyl, ethylsulfonyl, isopropylsulfonyl, n-butylsulfonyl, and the like;

"C₁-C₄ mono or dialkyl amino" includes amino, substituted with one or more C1-C4 alkyl groups as defined hereinabove, including: methylamino, ethylamino, n-butylamino, t-butylamino, dimethylamino, N,N-diethylamino, methyl-t-butylamino, and the like.

The R group or groups on the benzene ring can be present initially in the process, e.g. phenyl, methyl, methoxy, cyano, carbomethoxy, trifluoromethyl, (present as in the starting 6-nitrophenol 1 in Flow Chart A) or added later by a conventional reaction, e.g. chloro, as by chlorination, nitro by nitration, or created from a starting or added functional group present, e.g. converting a later added nitro to an amino group by catalytic reduction, then alkylating to a mono or dialkylamine. An amino group can be subjected to diazotization to a hydroxy group, which can be followed by methylation to a methoxy group. Similarly, a hydroxy group can be converted to a thiol by the analogous procedures described in J. Org. Chem. 31, pp 3980-3984 (1966) by Newman and Karnes, and J. Org. Chem. 31, pp 410 (1966) by Kwart, H. and Evans, E.S. The resulting thiol can be alkylated to alkylthio, which can be oxidized to the corresponding sulfoxide or sulfone. Preferred substituents are H, C₁-C₄ alkyl, C₁-C₄ alkoxy and phenyl. These reactions and sequences are conventional in the art and it will be obvious to one skilled in the art to modify the benzene ring to arrive at an R radical disclosed herein.

By the term "pharmaceutically acceptable salts and esters thereof" is meant salts and esters of the acid groups in the final molecule which can be used as part of the human drug delivery system and include the salts: sodium, potassium, calcium, ammonium, substituted ammonium, quaternary ammonium, and esters: ethyl ester, aceturate, besylate, edetate, phenpropionate, acetate, pamoate, and esters which serve as "prodrug" formulations which will hydrolyze in the body at physiological pH's to regenerate the acid, including pivaloylates, e.g. pivoxetil and pivoxil, and Kanebo esters, and the like. 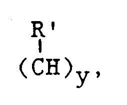
where y is 1-6, preferably 3, can contain at least one R' substituent as defined above, and can be, e.g., -CH₂-; -CH₂-CH₂-; -CH₂-CH₂-CH₂-; 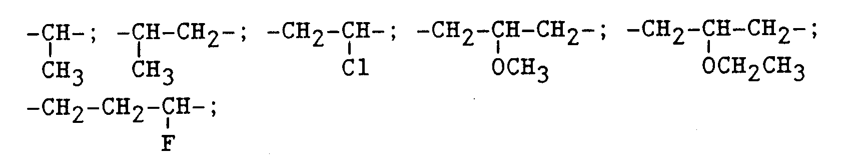
and the like.

An alkene bond can also be present in 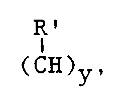
e.g., CH₂-CH = CH-; CH₂-CH = CH-CH₂-; -CH2-CH = CH-; -(CH₂)₃-CH = CH- and the like. 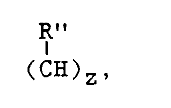
where z is 6-20, preferably 8-14, can contain at least one R" substituent as defined above, and can be completely alkyl; e.g., (CH₂)n-COOH, where n is 8-14 preferably and the like.

An alkene bond can also be present in 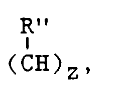
e.g., -(CH₂)₄-CH=CH-(CH₂)₄-, and the like.

Preferred is where one R' or R" is H and particularly preferred is where both 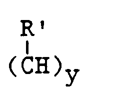
and 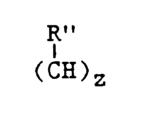
are alkyl.

Preferred compounds of the instant invention are given by the following formulas; 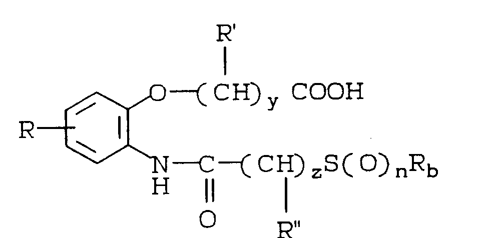
and, 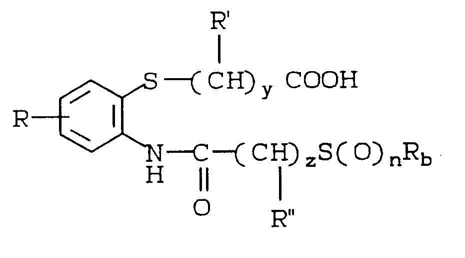
wherein R, R', R", Y, Z, R_{b} are defined above;
and particularly preferred are: 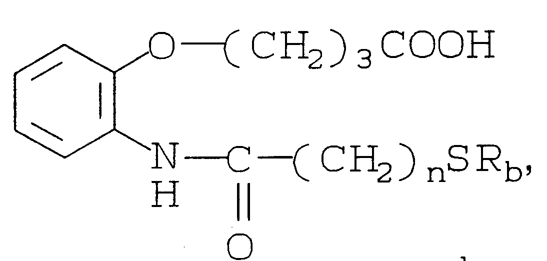 and 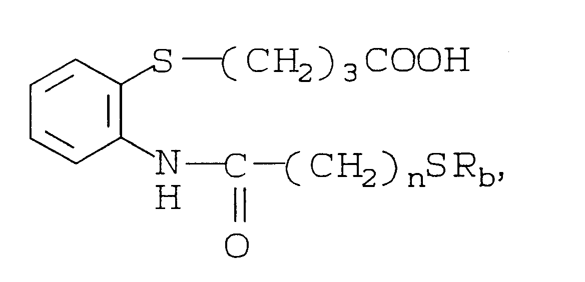
   where n is 8-14, and R_{b} is methyl, ethyl, cyclopropyl, isopropyl, n-propyl, t-butyl, phenyl or benzyl.

The compounds of the instant invention can be made by the procedures outlined in the following Flowcharts.

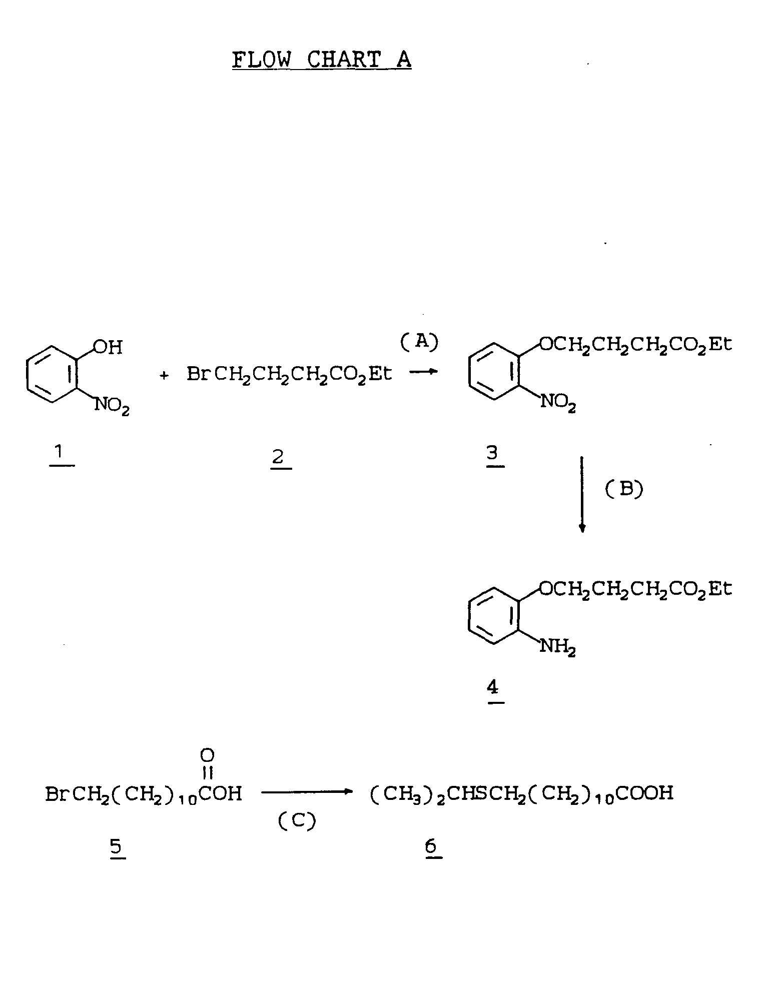

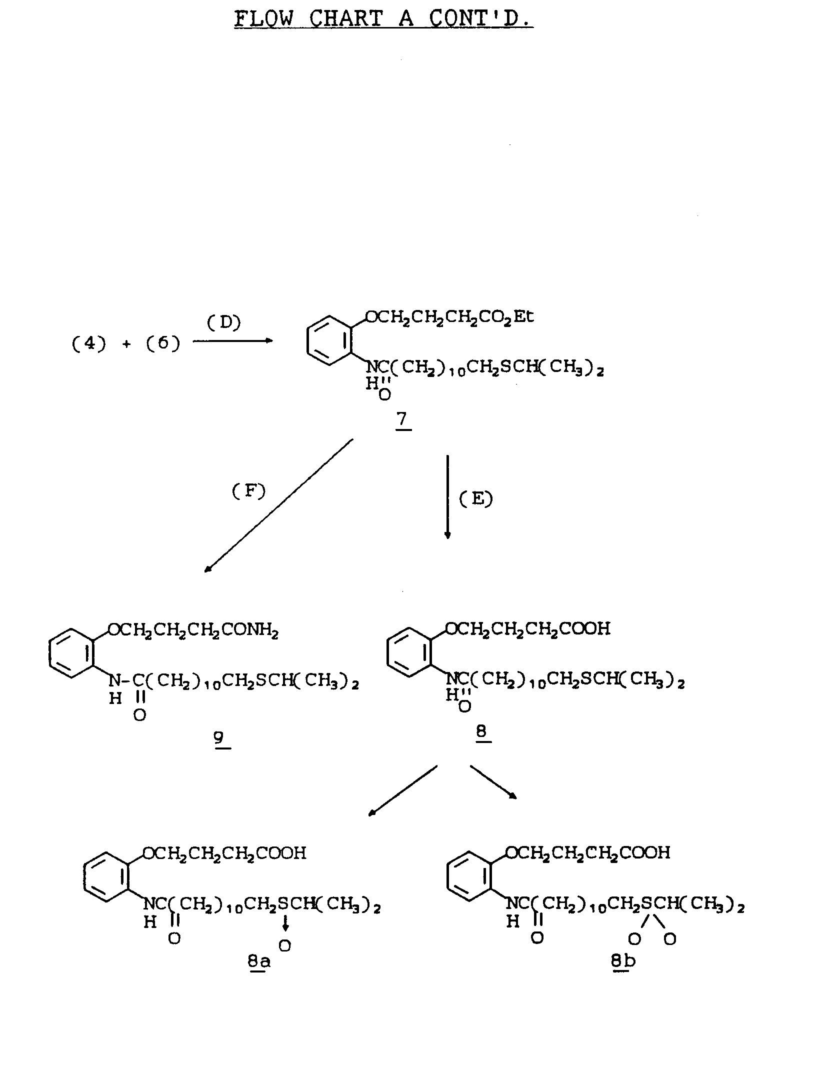

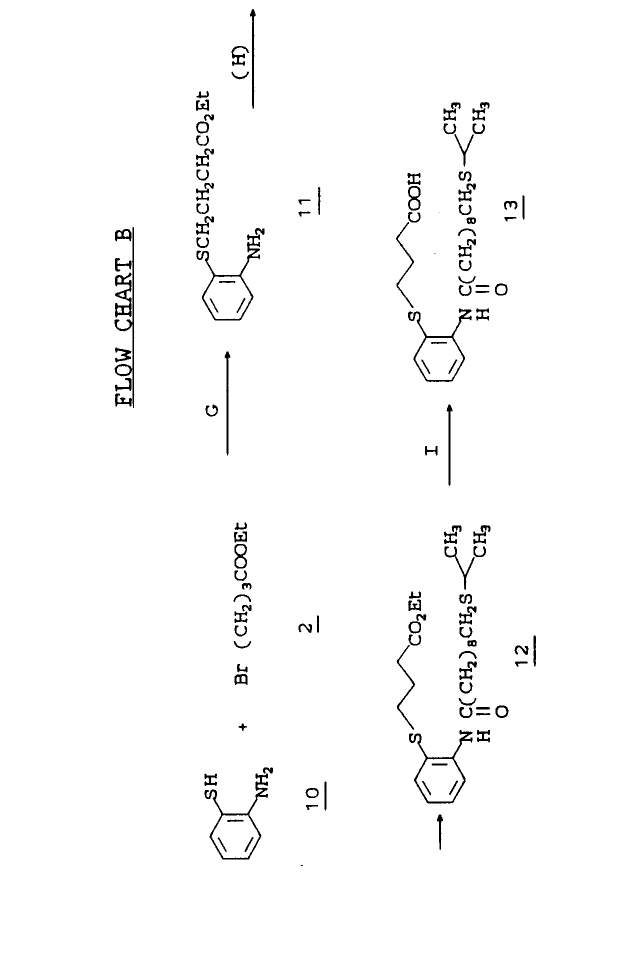

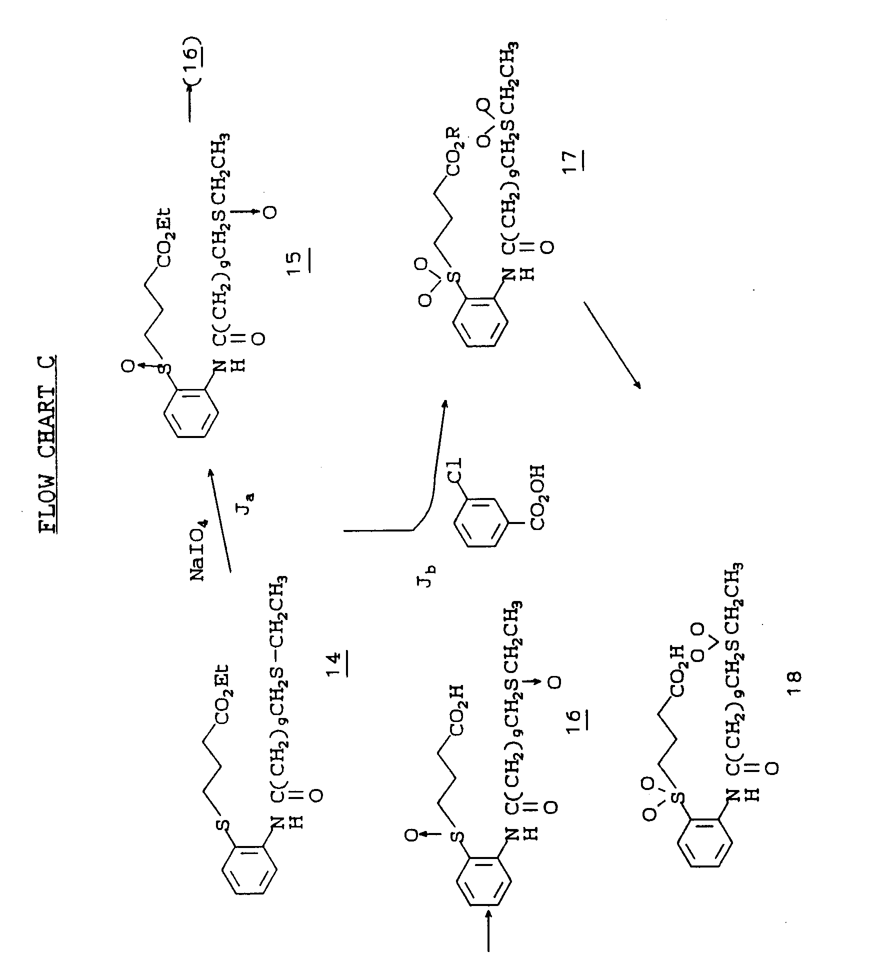

As seen in Flow Chart A, o-nitrophenol 1, ethyl 4-bromobutyrate 2 and anhydrous K₂C0₃ in e.g., dry acetone are heated at reflux e.g. for 12-100 hours, or stirred for an extended period of time at room temperature, under a nitrogen atmosphere to product ethyl 4-(2-nitrophenoxy) butyrate 3, in Step (A).

A solution of 3 in e.g., ethyl acetate is catalytically hydrogenated at room temperature under e.g. 40 psig of H₂ in the presence of a 5% Pd/C catalyst to yield ethyl 4-(2-aminophenoxy)butyrate 4 in Step (B).

Step (C) comprises reacting the 12-bromo dodecanoic acid 5 with isopropyl mercaptan in a suitable solvent, e.g., dimethoxyethane, at about 80-85 °C to obtain the acid̅ 6.

In Step (D) the mono acid 6 is reacted with the amine 4 and N̅'N'-dicyclohexylcarbodiimide (DCC) at e.g., room temperature in e.g., dry methylene chloride, optionally in the presence of 4-dimethylaminopyridine, to produce the amide 7.

In Step (E), the ether-amide 7 is de-esterified by e.g., 2.5 N NaOH in MeOH/H₂0 to yield the final product, monoacid 8.

The monoacid 8 can be treated with NalO₄ in (acetone/water) at room temperature for (4-24 hours) to produce the corresponding sulfoxide 8a. Additionally, 8 can be treated with meta-chlorobenzoic acid in (CH₂ C1₂) at a temperature of about 0° to 25° C for (1-24 hours) to produce the corresponding sulfone 8b.

In Step (F) the ester 7 is treated with ammonia in (methanol) at room temperature for, e.g., 1-7 days to produce the amide 9.

Flow Chart B illustrates the synthesis of the sulfur analogs of the invention compounds.

In Step G, the orthoaminothiophenol is reacted with the bromoester 2 under conditions similar to Step A to produce the thioether 11.

In Step (H), the thiother 11 is reacted with an alkylthioalkanoic acid under similar conditions using DCC analogously as in Step D to produce the acylated ester 12.

In Step I, the ester 12 is hydrolyzed to produce the free thio acid 13, which has the 5-a-reductase activity described herein.

As seen in Flow Chart C, the compound 13 can further be oxidized to the sulfoxide in Step Jₐ, starting with the thio compound 14 to produce the sulfoxide 15, which can be hydrolyzed, analogous to the conditions in Step I to produce the active acid 16.

In a similar manner, 14 can be converted to the sulfone-ester 17, which can then be hydrolyzed analogous to the conditions in Step I to the corresponding acid 18.

Alternatively, the sulfur in o-nitrobenzene thiol as a separate starting material analogous to 1, can be coupled to yield the ester alkylthio compound corresponding to 3 which can be oxidized to the corresponding sulfoxide or sulfone, then followed by reduction of the nitro group to the amino and then coupled with a suitable reagent e.g., 6, to yield the linear amide containing unoxidized sulfur analogous to 7. A further modification is where the sulfur acylating agent is first oxidized to the corresponding sulfoxide or sulfone, then coupled with the amino group of e.g., 11 to yield e.g. 13 containing only an oxidized sulfur in the amide chain.

It is obvious that other nitrophenols can be substituted for 1 in Flow Chart A to provide the scope of the compounds covered by this invention and include the following:
2-nitrophenol
2-nitro-6-methylphenol
2-nitro-5-methylphenol
2-nitro-4-methylphenol
2-nitro-3-methylphenol
2-nitro-4-phenylphenol
2-nitro-5-phenylphenol
2-nitro-4-chlorophenol
2-nitro-4-(trifluoromethyl)phenol
2-nitro-4-methoxyphenol
2-nitro-6-ethoxyphenol, and the like.

Starting materials for Flow Charts B and C in addition to 10 are commercially available and readily made by prior art procedures and include all of the above listed compounds where - SH is substituted for -OH, ortho to the nitro group.

Other starting materials for 2 in both Flow Charts A and B include the following:
Br-CH₂-COOMe,
Cl-CH₂CH₂CH₂COOCH(CH₃)₃,
Br-CH₂CH₂CH₂CH₂COOMe,
Br-CH₂ CH₂ CH₂ CH₂ CH₂ COOEt,
Br-CH₂CH₂CH₂CH₂CH₂CH₂COOCH₂CH₂CH₂CH₃,
Br-CH₂CH(CH₃)COOMe,
Br-CH₂CH(CH₃)CH₂COOEt,
Br-CH₂CH₂CH₂COOMe,
Br-CH₂CH(OCH₃)CH₂COOCH(CH₃)₂,
Cl-CH₂CH(OCH₂CH₃)CH₂COOMe,
Br-CH₂CH(F)CH₂COOMe,

and the like.

Other starting materials for the acid 6 to produce the acid for acylating the amino group in 4 or 11 include the following:
MeS-(CH₂)₆COOH,
MeS-(CH₂)₇COOH,
(CH₃)₂ CHS-(CH₂)₈ COOH,
EtS-(CH₂)₉COOH,
CH₃CH₂CH₂S(CH₂)₁₀COOH,
(CH3)2CHS(CH2)1 COOH,
MeS-(CH₂)₁₂COOH,
EtS-(CH₂)₁₃COOH,
CH₃CH₂CH₂S-(CH₂)₁₄COOH,
(CH₃)₂CHS-(CH₂)₁₅COOH,
CH₃(CH₂)₃S-(CH₂)₁₆COOH,
(CH₃)₂ CH-CH₂ S-(CH2)1 COOH,
CH₃-CH₂-CH₂-S-(CH₂)₁₈COOH,
(CH₃)₂CHS-(CH₂)₁₉ COOH,
EtS-(CH₂)₂₀COOH,
MeS-CH(CH₃)-(CH₂)_{1 0}COOH,
(CH₃)₂CHS-CH₂CH₂CH(CH₃)CH₂COOH,
MeS-CH₂CH₂CH-CH₂COOH CI
EtS-CH₂CH(OCH₃)(CH₂)₇COOH,
CH₃CH₂CH₂S-CH₂CH(OCH₂CH₃)CH₂CH₂COOH,
CH₃(CH₂)₇-S-CH₂-COOH
(CH₃)₂ CH(CH₂)₅ -S-CH₂-COOH
CH₃(CH₂)₉-S-CH₂-COOH
CH₃(CH₂)₁₁S-CH₂COOH, and the like.

Representative compounds of the instant invention include, but are not limited to:
4-(2-(20-lsopropylthioeicosanoylamino)phenoxy)butyric acid;
4-(2-(19-Methylthiononadecanoylamino)phenoxy)butyric acid;
4-(2-(18-Ethylthioloctadecanoylamino)phenoxy)butyric acid;
4-(2-(17-Isopropylthioheptadecanoylamino)phenoxy)butyric acid;
4-(2-(16-Methylthiohexadecanoylamino)phenoxy)butyric acid;
4-(2-(15-Methylsulfinylpentadecanoylamino)phenoxy)butyric acid;
4-(2-(14-Methylsulfinyltetradecanoylamino)phenoxy)butyric acid;
4-(2-(13-n-Propylthiotridecanoylamino)phenoxy)butyric acid;
4-(2-(12-n-Butylsulfinyldodecanoylamino)phenoxy)butyric acid;
4-(2-(11-sec-Butylthioundecanoylamino)phenoxy)butyric acid;
4-(2-(10-phenylthiodecanoylamino)phenoxy)-butyric acid;
4-(2-(10-benzylthiodecanoylamino)phenoxy)-butyric acid;
4-(2-(10-iso-Butylsulfonyldecanoylamino)phenoxy)-butyric acid;
4-(2-(9-t-Butylthiononanoylamino)phenoxy)butyric acid;
4-(2-(8-Ethylsulfonyloctanoamino)phenoxy)butyric acid;
4-(2-(7-Isopropylthioheptanoylamino)phenoxy)butyric acid;
4-(2-(6-Methylthiohexanoylamino)butyric acid;
4-(2-(20-Ethylsulfonyleicosanoylamino)phenylthio)-butyric acid;
4-(2-(19-Isopropylthiononadecanoylamino)phenylthio)-butyric acid;
4-(2-(18-Methylthiooctadecanoylamino)phenyithio)-butyric acid;
4-(2-(17-Ethylthioheptadecanoylamino)phenylthio)-butyric acid;
4-(2-(16-lsopropylhexadecanoylamino)phenylthio)-butyric acid;
4-(2-(15-Methylthiopentadecanoylamino)phenylthio)-butyric acid;
4-(2-(14-Methylsulfinyltetradecanoylamino)phenylthiobutyric acid;
4-(2-(13-Methylsulfonyltridecanoylamino)butyric acid;
4-(2-(12-n-Propylthiododecanoylamino)phenylthio)-butyric acid;
4-(2-(11-n-Butylsulfinylundecanoylamino)phenylthio)-butyric acid;
4-(2-(10-sec-Butylthiodecanoylamino)phenylthio)-butyric acid;
4-(2-(10-phenylthiodecanoylamino)phenylthio)-butyric acid;
4-(2-(10-benzylthiodecanoylamino)phenylthio)-butyric acid;
4-(2-(9-iso-Butylsulfonylnonanoylamino)phenylthio)-butyric acid;
4-(2-(8-t-Butylthiooctanoylamino)phenylthio)butyric acid;
4-(2-(7-Ethylsulfinylheptanoylamino)phenylthio butyric acid;
4-(2-(6-lsopropylthiohexanoylamino)phenylthio butyric acid;
3-(2-(16-Methylsulfinylhexadecanoylamino)phenoxy)-propionic acid;
4-(2-(15-Methylsulfonylisohexadecanoylamino)phenoxy)-butyric acid;
3-(2-(14-n-Propylthiotetradecanoylamino)phenoxy)-isobutyric acid;
5-(2-(13-n-Butylsulfinyltridecanoylamino)phenoxy)-valeric acid;
5-(2-(12-sec-Butylthiododecanoylamino)phenoxy)-valeric acid;
5-(2-(11-iso-Butylsulfonylisododecanoylamino)-valeric acid;
5-(2-(11-t-Butylthioundecanoylamino)valeric acid;
5-(2-(10-Ethylsulfinyldecanoylamino)valeric acid;
5-(2-(9-lsopropylthiononanoylamino)phenoxy)valeric acid;
6-(2-(9-Methylthiononanoylamino)phenoxy)caproic acid;
6-(2-(8-Ethylthiooctanoylamino)phenoxy)caproic acid;
6-(2-(7-Isopropylthioisooctanoylamino)phenoxy)caproic acid;
7-(2-(7-Methylheptanoylamino)phenoxy)enanthic acid;
7-(2-(6-Methylsulfinylhexanoylamino)phenoxy)enanthic acid;
7-(2-(5-Methylsulfonylisohexanoylamino)phenoxy)-enanthic acid;
2-(2-(12-n-Propylthiododecanoylamino)phenoxy))acetic acid;
2-(2-(11-n-Butylsulfinylundecanoylamino)phenoxy)acetic acid;
2-(2-(10-sec-Butylthiodecanoylamino)phenoxy)acetic acid;
3-(2-(9-iso-Butylsulfonylnonanoylamino)propionic acid;
3-(2-(12-t-Butylthiododecanoylamino)phenylthio)-propionic acid;
3-(2-(11-Ethylsulfinylundecanoylamino)phenylthio)-propionic acid;
4-(2-(11-Isopropylthioundecanoylamino)phenylthio)-butyric acid;
4-(2-(11-Methylthioundecanoylamino)-4-methyl-thiophenoxy)butyric acid;
4-(2-(12-Ethylthiododecanoylamino)phenylthio)-butyric acid;
5-(2-(11-Isopropylthioundecanoylamino)phenylthio)-valeric acid;
5-(2-(10-Methylthiodecanoylamino)phenylthio)valeric acid;
5-(2-(9-Methylsulfinylnonanoylamino)phenylthio)-valeric acid;
5-(2-(12-Methylsulfonyldodecanoylamino)phenylthio)-valeric acid;
5-(2-(11-n-Propylthiodecanoylamino)phenylthio)-valeric acid;
5-(2-(10-n-Butylsulfinyldecanoylamino)phenylthio)-valeric acid;
6-(2-(9-sec-Butylthiononanoylamino)phenoxy)caproic acid;
6-(2-(12-iso-Butylsulfonyldodecanoylamino)phenylthio) caproic acid;
6-(2-(11-t-Butylthioundecanoylamino)phenylthio)-caproic acid;
7-(2-(11-Ethylsulfinylundecanoylamino)-3-methylphenylthio)enanthic acid;
7-(2-(11-Isopropylthioundecanoylamino)-4-methylphenylthio)enanthic acid;
7-(2-(12-Methylthiododecanoylamino)phenoxy)enanthic acid;
4-(2-(11-Phenylthioundecanoylamino)4-methyl-phenoxy)-butyric acid;
4-(2-(10-Benzylthiodecanoylamino)3-methylphenoxy)-butyric acid;
4-(2-(9-Methylthiononanoylamino)5-methylphenoxy)-butyric acid;
4-(2-(12-Methylsulfinyldodecanoylamino)6-methylphenoxy)butyric acid;
4-(2-(11-Methylsulfonyldecanoylamino)3-phenylthio) butyric acid;
4-(2-(10-n-Propylthiodecanoylamino)4-methylphenoxy)-butyric acid;
4-(2-(9-n-Butylsulfinylnonanoylamino)5-fluoromethylphenylthio)butyric acid;
4-(2-(12-sec-Butylthiododecanoylamino)6-methylphenoxy)butyric acid;
5-(2-(11-iso-Butylsulfonylundecanoylamino)-3-methylphenylthio)valeric acid;
4-(2-(11-t-Butylthioundecanoylamino)-3-methylsulfonylphenoxy)butyric acid;
4-(2-(11-Ethylsulfinylundecanoylamino)-4-methylsulfonylphenylthio)butyricacid;
4-(2-(12-Isopropylthiododecanoylamino)5-ethylphenoxy)butyric acid;
4-(2-(11-Methylthioundecanoylamino)4-phenylphenoxy)-butyric acid;
4-(2-(10-Ethylthiodecanoylamino)-3,5-dimethylphenoxy)-butyric acid;
4-(2-(9-lsopropylthiononanoylaminc)-4-fluoro-phenoxy)-butyric acid;
4-(2-(12-Methylthiododecanoylamino)-5-trifluoromethylphenoxy)butyric acid;

Representative examples of preferred compounds produced by this process include:
4-(2-(11-Isopropylthio)undecanoylamino)phenoxy)butyric acid,
4-(2-(11-Ethylthio)undecanoylamino)phenylthio)butyric acid,
4-(2-(9-lsopropylthio)nonanoylamino)phenoxy)butyric acid,
4-(2-(10-Isopropylthio)decanoylamino)phenoxy)butyric acid,
4-(2-(12-Isopropylthio)dodecanoylamino)phenoxy)butyric acid,
4-(2-(13-Butylthio)tridecanoylamino)phenoxy)butyric acid,
4-(2-(15-t-Butylthio)pentadecanoylamino)phenoxy)butyric acid,
5-(2-(11-Isopropylthio)undecanoylamino)phenoxy)valeric acid,
4-(2-(11-Ethylsulfinyl)undecanoylamino)-phenoxy)butyric acid,
4-(2-(11-Isopropylsulfonyl)undecanoylamino)-4-methylphenoxy)butyric acid,
4-(2-(11-Ethylsulfinyl)undecanoylamino)-5-methylphenoxy)butyric acid.

The compounds of the present invention, prepared in accordance with the methods described above, are, as already described, can reduce dihydrotestosterone lends by virtue of their ability to specifically inhibit testosterone-5a-reductase.

Accordingly, the present invention is also particularly concerned with providing a method of treating the hyperandrogenic conditions of acne vulgaris, seborrhea, and female hirsutism by topical administration, and a method of treating all of the above conditions as well as benign prostatic hypertrophy, by oral or parenteral administration, of the novel compounds of the present invention.

The present invention is thus also concerned with providing suitable topical, oral and parenteral pharmaceutical formulations for use in the novel methods of treatment of the present invention.

The compositions containing the compounds of the present invention as the active ingredient for use in the treatment of 5-reductase disorders including benign prostatic hyperplasia can be administered in a wide variety of therapeutic dosage forms in conventional vehicles for systemic administration, as, for example, by oral administration in the form of tablets, capsules, solutions, or suspensions, of by intravenous injection. The daily dosage of the products may be varied over a wide range varying from 50 to 2,000 mg. The compositions are preferably provided in the form of scored tablets containing 5, 10, 25, 50, 100, 150, 250, and 500 milligrams of the active ingredient for the symptomatic adjustment of the dosage to the patient to be treated. An effective amount of the drug is ordinarily supplied at a dosage level of from about 0.01 mg to about 50 mg/kg of body weight per day. Preferably the range is from about 0.1 mg to 7 mg/kg of body weight per day. These dosages are well below the toxic dose of the product. Capsules containing the product of this invention can be prepared by mixing an active compound of the present invention with lactose and magnesium stearate, calcium stearate, starch, talc, or other carriers, and placing the mixture in gelating capsule. Tablets may be prepared by mixing the active ingredient with conventional tableting ingredients such as calcium phosphate, lactose, corn starch or magnesium stearate. The liquid forms in suitably flavored suspending or dispersing agents such as the synthetic and natural gums, for example, tragacanth, acacia, methylcellulose and the like. Other dispersing agents which may be employed include glycerin and the like. For parenteral administration, sterile suspensions and solutions are desired. Isotonic preparations which generally contain suitable preservative are employed when intravenous administration is desired.

For the treatment of acne vulgaris, seborrhea, female hirsutism, the compounds of the present invention are administered in the formula of pharmaceutical composition comprising the active compound in combination with a pharmacologically acceptable carrier adapted for topical administration. These topical pharmaceutical compositions may be in the form of a cream, ointment, gel or aerosol formulation adapted for application to the skin. These topical pharmaceutical compositions containing the compounds of the present invention ordinarily include about 0.1% to 15%, preferably about 5%, of the active compounds, in admixture with about 95% of vehicle.

The method of preparing the novel compounds of the present invention, already described above in general terms, may be further illustrated by the following examples which should not be construed as being limitations on the scope or spirit of the instant invention.

### EXAMPLE 1

### Preparation of 4-(2-(11-carboxyundecanoylamino)-phenoxy)butyric acid

### Step A: Ethyl 4-(2-nitrophenoxy)butyrate(3)

To a stirred solution of 2-nitrophenol (1.4 g, 10 mM) and ethyl 4-bromobutyrate (2.1 g, 1.57 mL, 11 mM) in 35 mL of dry acetone is added 2 g (14.5 mM) of anhydrous, ground potassium carbonate. The resultant colored mixture is then heated under a nitrogen atmosphere at gentle reflux until the color due to the phenol anion has dissipated and a yellow mixture remains. Concentration of the cooled and filtered mixture yields an oil which on flash chromatography (silica gel, ethyl acetate/hexane or methylene chloride as eluant) yields 2.4 g (96% yield) of the title compound (3) as an oily liquid. When substituted ortho-nitrophenols are used in place of 2-nitrophenol in the above example, the corresponding substituted 2-nitrophenoxybutyrate is obtained. Likewise, when ethyl 4-bromobutyrate is replaced by other halo esters. the corresponding 2-nitrophenoxyalkanoate is obtained.

### Step B: Ethyl 4-(2-aminophenoxy)butyrate (4)

A solution of 3 (1.27 g, 5.0 mM) in 15 mL ethyl acetate containing 200 mg of 5% palladium on carbon is reacted in a hydrogen atmosphere (40 psig.) at room temperature until hydrogen uptake ceases. The mixture is then filtered and concentrated in vacuo to yield 1.0 + g of (4) as an oil/low melting solid.

### Step C: 12-(Isopropylthio)dodecanoic acid (6)

A mixture of 12-bromododecanoic acid (5) (0.558 g, 2.0 mM) and sodium isopropylthiolate (1.1 g, 11.2 mM) in 1,2-dimethoxyethane (50 mL) was deaerated (N₂), heated to 85°C (bath temperature), and kept at this temperature for 72 hours. The cooled mixture was filtered, the collected solid dissolved in water and filtered. The stirred solution was acidified with dilute hydrochloric acid, aged, filtered, the solid washed well with water and dried. There was obtained product 6 (0.54 g) as a white solid.

When other halo-acids are used in place of 12-bromododecanoic acid in the above example, the corresponding (isopropylthio)-acid is obtained.

Likewise, when other mercaptan salts are used in place of sodium isopropylthiolate in the above example, the corresponding (alkylthio)alkanoic acids are obtained.

Representative of, but not limited to, the acids obtained by this procedure are:
8-(lsopropylthio)octanoic acid
10-(Isopropylthio)decanoic acid
10-(Ethylthio)decanoic acid
11-(t-Butylthio)undecanoic acid
14-(n-Propylthio)tetradecanoic acid
9-(Methylthio)nonanoic acid

### Step D: Ethyl 4-(2-(12-(Isopropylthio)dodecanoylamino)-phenoxy)-butyrate (7)

To a solution of (4) (0.25 g, 1.14 mM) and (6) (0.274 g, 1.0 mM) in dry methylene chloride (10 mL) at room temperature was added 4-dimethylaminopyridine (0.122 g, 1.0 mM) followed within one minute by a solution of N,N'-dicyclohexylcarbodiimide (0.22 g, 1.06 mM) in methylene chloride (1 mL), 3X1 mL rinses with methylene chloride. After 2 days, the filtered mixture was concentrated in vacuo and the residue flash chromatographed on silica gel using 15-20% ethyl acetate in hexane as eluant to give product 7 (0.22 g) as an oil that solidified readily in a short time.

### Step E 4-(2-(12-(Isopropylthio)dodecanoylamino) phenoxy)-butyric acid (8)

A stirred solution of ester (7) (0.124 g, 0.258 mM) in methanol (10 mL) was treated at room temperature under a nitrogen atmosphere with 2.5N sodium hydroxide solution (0.6 mL). Methanol (2X2 mL) was used to clear the mixture, and the reaction allowed to continue until TLC analysis showed no ester remained. The filtered mixture was concentrated in vacuo and the residue obtained stirred with water (30 mL). After aging, the mixture was filtered (The cake is the sodium salt of the product (100 mg), and the stirred filtrate acidified with dilute hydrochloric acid, aged, filtered, washed with water and dried to give product 8 (0.02 g) as a white solid. M.P. 82-84 C, with softening from 66 C.

Treatment of 8 with Nal0₄ as in Step Jₐ will produce the corresponding sulfoxide, and treatment of 8 with m-chloroperbenzoic acid with Step J_{b} will produce the corresponding sulfone.

### Step F: 4-(2-(12-(Isopropylthio)dodecanoylamino) phenoxy)-butyramide (9)

To a stirred solution of (7) (20 mg, 0.041 mM) in methanol (10 mL) is added methanol saturated with ammonia (5 mL) and the stoppered mixture allowed to stir at ambient temperatures until TLC analysis shows little or no (D) remains. Concentration of the reaction mixture followed by preparative thin layer chromatography (silica gel; 3% methanol/methylene chloride as eluant) yields product 9 (11 mg) as a waxy solid.

### Step G: Ethyl 4-(2-Amino-phenylthio)butyrate (11)

To a stirred deaerated (N₂) solution of 2-aminothiophenol (10) (1.25 g., 10mM) and ethyl 4-bromobutyrate (2.14g., 11mM) in 40mL. of dry 1,2-dimethoxyethane is added 8.3g. of solid ground anhydrous potassium carbonate, the resultant mixture deaerated 3X under nitrogen and allowed to stir at room temperature until TLC analysis indicates the reaction is complete. The filtered mixture is then concentrated and the residue flash chromatographed on silica gel (85g.) using 15% ethyl acetate/hexane as eluant to give 1.8g. of (11) as a pale tan oil.

### Step H: Ethyl 4-(2-(10-(Isopropylthio)decanoylamino)-phenylthio)butyrate (12)

When (11) and 10-(lsopropylthio)decanoic are reacted together analogously as per the conditions in Step (D) above, the title compound 12 is obtained.

### Step I: 4-(2-(10-(Isopropylthio)decanoylamino) phenylthio)-butyric acid (13)

When (12) is hydrolyzed as per the conditions of Step (E) the title compound 13 is obtained.

### Step J: 4-(2-(11-(ethylsulfinyl)undecanoylamino) phenoxy)-butyric acid

When the subject esters or acids are treated with sodium metaperiodate (Step Jₐ) in a suitable solvent (e.g. acetone/water) the corresponding sulfoxides are obtained. Treatment with meta-chloroperbenzoic acid (Step J_{b}) yields the corresponding sulfones. For example, when ethyl 4-(2-(11-(ethylthio)undecanoylamino)-phenoxy)-butyrate 14 (0.045 g, 0.1 mM) in acetone (10 mL) is reacted with sodium metaperiodate (0.072 g, 0.33 mM) in water (2 mL) at room temperature the corresponding sulfoxide 15 is obtained. Hydrolysis as per Step (E) yields product 16 as an off-white solid. Additionally, treatment of 14 with m-chloroperbenzoic acid will produce the sulfone 17, which yields the acid 18 upon hydrolysis using the hydrolysis conditions of Step E.

Compounds (3)-(16) all had NMR and Mass Spectral data consistent with their assigned molecular structures.

## Claims

1. A compound of the structure: 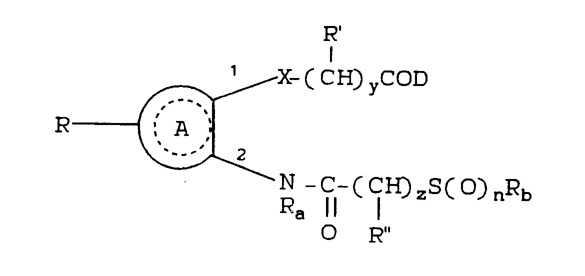
wherein
A is a 1,2-disubstituted aromatic ring;
D is OH, NH₂, NHR_{b}, OR_{c};
X is O, S, SO, or S0₂;
R is H,
C₁-C₄ alkyl,
phenyl or substituted phenyl,
halo,
haloalkyl,
hydroxy,
carboxy,
cyano,
C₁-C₄ alkoxy,
C₁ -C₄ alkylthio,
C₁-C₄ alkylsulfinyl,
C₁ -C₄ alkylsulfonyl,
nitro,
amino,
C₁-C₄ mono or di-alkylamino;
R' and R" are independently
H,
halo,
C₁-C₄ alkyl or C₁-C₄ alkoxy,
amino, or oxo, where CH-R' or CH-R"
in the formula become -C = O;
Rₐ is H, C₁-C₄ alkyl;
R_{b}, Rₑ are independently, C₁-C₁₂ alkyl, phenyl, phenyl-C₁-C₄ alkyl;
n is 0-2;
y is 1-6;
z is 6-20; and
wherein 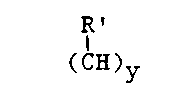
and 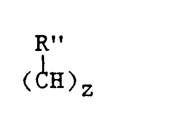
can independently represent substituted or unsubstituted alkyl or alkenyl radicals containing at least one alkene bond;
and pharmaceutically acceptable salts and esters thereof.

2. The compound of claim 1 wherein X is O.

3. The compound of claim 1 wherein R is H.

4. The compound of claim 1 wherein one of R' or R" are H.

5. The compound of claim 1 wherein y is 3.

6. The compound of claim 1 wherein (Z) is 8-14.

7. The compound of claim 1 wherein both R'(CH)y and R"(CH)₂ are alkyl.

8. The compound of claim 1 of the structure: 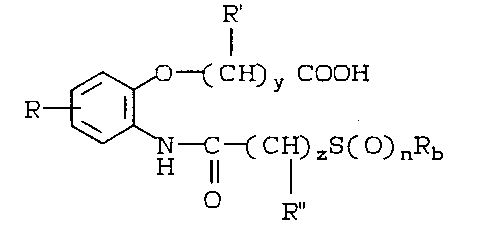
wherein
R is H, C₁-C₄ alkyl, phenyl or substituted phenyl, halo, haloalkyl, hydroxy, C₁-C₄ alkoxy, C₁-C₄ alkylthio, C₁-C₄ alkylsulfinyl, C₁-C₄ alkylsulfonyl, amino, C₁-C₄ mono or di-alkylamino;
R' and R" are independently H, halo, C₁-C₄ alkyl or C₁-C₄ alkoxy, amino, or oxo, where CH-R' or CH-R" in the formula become -C = O;
n is 0-1;
y is 1-6; z is 6-20;
and wherein R_{b} is C₁ -C₄ alkyl and 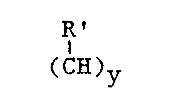
and 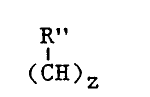
can independently represent substituted or unsubstituted alkyl or alkenyl radicals containing at least one alkene bond; and pharmaceutically acceptable salts and esters thereof.

9. The use of a compound of claim 1 for the manufacture of a medicament for treating the hyperandrogenic condition of acne vulgaris, seborrhea, female hirsutism, and benign prostatic hypertrophy.

10. A pharmaceutical composition comprising a pharmaceutically acceptable carrier and a therapeutically effective amount of a compound according to Claim 1.
